# EUROPEAN PATENT APPLICATION

(11) **EP 4 427 755 A1**
(43) Date of publication of application: **11.09.2024**
(21) Application number: 22890434.8
(22) Date of filing: 04.11.2022
(51) Int. Cl.: A61K 35/74, A61K 45/06, A61P 35/00, C07K 16/28, A61K 39/00

(54) **PHARMACEUTICAL COMPOSITION COMPRISING SALMONELLA STRAIN AND IMMUNE CHECKPOINT INHIBITOR AS ACTIVE INGREDIENT FOR PREVENTION OR TREATMENT OF CANCER**

(30) Priority: 05.11.2021 KR 20210151691
(71) Applicant: Cncure Biotech Inc., Jeollanam-do 58128 (KR)
(72) Inventor: MIN, Jung-Joon, Gwangju 61461 (KR); HONG, Yeongjin, Gwangju 61682 (KR); YOU, Sung-Hwan, Gwangju 61419 (KR); HUY, Nguyen Dinh, Hwasun-gun Jeollanam-do 58116 (KR)
(74) Representative: Latscha Schöllhorn Partner AG
(86) International application number: PCT/KR2022/017209
(87) International publication number: WO 2023/080703

(57) **Abstract**

The present invention relates to a pharmaceutical composition for preventing or treating cancer comprising a *Salmonella* strain and an immune checkpoint inhibitor as active ingredients. The composition of the present invention may be useful as a prophylactic or therapeutic composition of improving the survival rate by significantly reducing the tumor size by co-administering bacteria and an immune checkpoint inhibitor in cancer, especially a type of cancer that is resistant and difficult to treat by a single anticancer therapy.

## Description

### Technical Field

The present invention relates to a pharmaceutical composition for preventing or treating cancer comprising a *Salmonella* strain and an immune checkpoint inhibitor as active ingredients.

### Background Art

With the advancement of immunology, immune checkpoint inhibitors have been developed, opening a new era in cancer treatment. However, since patients who respond effectively to immune checkpoint inhibitors differ depending on the type of cancer, and immune checkpoint inhibitors are effective in less than 30% of patients, many studies have been conducted to improve the therapeutic effect of immune checkpoint inhibitors. Thereamong, bacteria have received considerable attention as therapeutic agents. Bacterial monotherapy may not provide complete treatment, but studies thereon have been conducted to increase the therapeutic effect thereof by changing to anticancer drugs and by using antitumor genes or immunogenic antigens alone or in combination.

When cancer occurs in an individual, blood vessel formation and cell growth proceed at a very rapid rate in the body, and an oxygen-deficient environment may be created in the cancer tissue due to incomplete blood vessel formation. Thus, the cancer tissue may be very suitable for the growth of anaerobic bacteria such as *Salmonella* sp. strains or *E. coli.* Accordingly, current cancer therapies that use bacteria capable of targeting cancer, such as *Salmonella* sp. strains and *Clostridium* sp. strains, rely on the functions of specific bacteria that target solid tumors and can proliferate within the tumors. However, when an oncolytic protein or a reporter protein is introduced into the bacteria and the transformed bacteria are administered to an individual, it is possible to treat cancer by specifically identifying cancer tissue or minimizing side effects that are toxic to normal cells.

Therefore, the present invention has been made in order to solve the problems associated with the development of a prophylactic or therapeutic agent for solid cancer, and relates to a pharmaceutical composition for preventing or treating cancer comprising a *Salmonella* strain and an immune checkpoint inhibitor as active ingredients. The pharmaceutical composition of the present invention has the effect of improving the survival rate in the *in vivo* system, and thus is expected to be widely used in the field of cancer treatment.

### DISCLOSURE

### Technical Problem

The present inventors have made extensive research efforts to discover a composition for the highly reliable prevention and treatment of a cancer that is resistant and difficult to treat by a single anticancer therapy and for which there is no effective therapeutic method. As a result, the present inventors have found that tumor growth is significantly inhibited and reduced by a combination therapy of a *Salmonella* strain that selectively acts on cancer and an immune checkpoint inhibitor including PD-L1. As a result, the present inventors have discovered a new and efficient therapeutic method and composition for cancer, thereby completing the present invention.

Therefore, an object of the present invention is to provide a pharmaceutical composition for preventing or treating cancer comprising a *Salmonella* strain and an immune checkpoint inhibitor as active ingredients.

Other objects and advantages of the present invention will be more apparent from the following detailed description, the appended claims and the accompanying drawings.

However, objects to be achieved by the present invention are not limited to the objects mentioned above, and other objects not mentioned above can be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the art to which the present invention pertains.

Throughout the present specification, it is to be understood that when any part is referred to as "comprising" any component, it does not exclude other components, but may further comprise other components, unless otherwise specified.

According to one aspect of the present invention, the present invention provides a pharmaceutical composition for preventing or treating cancer, comprising a *Salmonella* strain and an immune checkpoint inhibitor as active ingredients.

The present inventors have made extensive research efforts to discover a composition for the highly reliable prevention and treatment of a cancer that is resistant and difficult to treat by a single anticancer therapy and for which there is no effective therapeutic method. As a result, the present inventors have found that tumor growth is significantly inhibited and reduced by a combination therapy of a *Salmonella* strain that selectively acts on cancer and an immune checkpoint inhibitor including PD-L1. As a result, the present inventors have discovered a new and efficient therapeutic method and composition for cancer, thereby completing the present invention.

In the present specification, the term "*Salmonella*" refers to a type of Proteobacteria belonging to the genus *Salmonella* genus of the family *Enterobacteriaceae. Salmonella* is a rod-shaped bacterium with a diameter of about 0.7 to 1.5 µm and a length of about 2 to 5 µm, and mainly lives in the digestive tracts of humans and animals.

In the present specification, the term "immune checkpoint" refers to an intracellular signaling system that maintains self-tolerance and protects tissues from excessive immune responses that cause damage. Immune checkpoint proteins are cell membrane proteins that regulate immune checkpoint and are able to inhibit differentiation, proliferation, and activity of immune cells. Specifically, these immune checkpoint proteins are expressed in activated T cells and function to reduce T cell proliferation, cytokine secretion, and cytotoxicity, and inhibit excessive activity of T cells. Some immune checkpoints act as one of the main mechanisms by which tumor cells evade the patient's immune response. Accordingly, the term "composition for immune checkpoint inhibition" or "immune checkpoint inhibitor" refers to a type of cancer immunotherapy that is the principle of inducing the body's immune system to selectively attack cancer cells. The "immune checkpoint inhibitor" refers to an anticancer component or anticancer adjuvant component that blocks the expression or activity of an immune checkpoint protein, thereby enhancing T cell activity, thus promoting an antitumor immune response.

In the present specification, the term "cancer" is characterized by unregulated cell growth. This abnormal cell growth results in the formation of a cell mass called a tumor, which infiltrates into surrounding tissues and, in severe cases, metastasize into other organs of the body. Academically, cancer is also called neoplasia. Cancer is an intractable chronic disease that is not fundamentally cured in many cases even if it is treated by surgery, radiotherapy, and chemotherapy, causes pain to the patient, and ultimately leads to death. Cancer is caused by various factors which are divided into internal factors and external factors. Although a mechanism by which normal cells are transformed into cancer cells has not been clearly found, it is known that a significant number of cancers are caused by external factors such as environmental factors. The internal factors include genetic factors, immunological factors, and the like, and the external factors include chemical substances, radiations, viruses, and the like. Genes involved in cancer development include oncogenes and tumor suppressor genes, and cancer develops when the balance between these genes is broken by the above-described internal or external factors. Examples of cancer to be prevented, ameliorated or treated in the present invention include, but are not limited to, melanoma, fallopian tube cancer, brain cancer, small intestine cancer, esophagus cancer, lymph node cancer, gallbladder cancer, blood cancer, thyroid cancer, endocrine cancer, oral cancer, liver cancer, biliary tract cancer, colorectal cancer, rectal cancer, cervical cancer, ovarian cancer, kidney cancer, gastric cancer, duodenum cancer, prostate cancer, breast cancer, brain tumor, lung cancer, undifferentiated thyroid cancer, uterine cancer, colon cancer, bladder cancer, ureteral cancer, pancreatic cancer, bone/soft tissue sarcoma, skin cancer, non-Hodgkin lymphoma, Hodgkin lymphoma, multiple myeloma, leukemia, myelodysplastic syndrome, acute lymphoblastic leukemia, acute myeloid leukemia, chronic lymphocytic leukemia, chronic myeloid leukemia, and solitary myeloma

In the present specification, the term "prevention" means inhibiting the occurrence of a disorder or a disease in a subject who has never been diagnosed as having the disorder or disease, but is likely to suffer from such disorder or disease.

In the present specification, the term "treatment" refers to any action that alleviates symptoms caused by cancer or benefits an individual, by using the active ingredients of the present invention, and refers to an approach for obtaining beneficial or desired results, including clinical results. Beneficial or desired clinical results may include, but are not necessarily limited to, alleviation or amelioration of one or more symptoms or conditions; diminishment of extent of disease; stabilization of the state of disease; prevention of development of disease; prevention of spread of disease; delay or slowing of disease progression, delay or slowing of disease onset, amelioration or palliation of the disease state; and remission (whether partial or total), whether detectable or undetectable. "Treatment" can also mean prolonging survival of a patient beyond that expected in the absence of treatment. In addition, "treatment" can also mean inhibiting the progression of disease or slowing the progression of disease temporarily, More preferably, it involves halting the progression of the disease permanently. As will be understood by a skilled person, results may not be beneficial or desirable if, while improving a specific disease state, the treatment results in adverse effects on the patient treated that outweigh any benefits effected by the treatment.

Thus, the composition of the present invention may serve as a therapeutic composition for the disease alone, or may be administered in combination with other anticancer agents and applied as a therapeutic aid for the disease. Accordingly, as used in the present specification, the term "treatment" or "therapeutic agent" encompasses the meaning of "treatment aid" or "therapeutic aid agent".

In the present invention, for use, the pharmaceutical composition may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition of the present invention may further comprise pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preserving agent, a pain-relieving agent, a solubilizer, an isotonic agent, a stabilizer, and the like may be mixed and used for injection; and a base, an excipient, a lubricant, a preservative, and the like may be used for topical administration. The pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like. Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further comprise a filler, an anticoagulant, a lubricant, a wetting agent, a fragrance, an emulsifier, a preservative, or the like.

In the present specification, the term "administration" or "administering" means administering a therapeutically effective amount of the composition of the present invention directly to a subject so that the same amount is formed in the subject's body. The term includes introducing the composition of the present invention into a patient by any suitable method. The composition of the present invention may be administered by any general route, as long as it can reach a target tissue. Examples of the administration route include, but are not limited to, oral administration, intraperitoneal administration, intravascular administration, intramuscular administration, subcutaneous administration, intradermal administration, intranasal administration, intrapulmonary administration, intrarectal administration, intracavitary administration, intraperitoneal administration, and intradural administration. In the present invention, the effective amount may be adjusted depending on various factors, including the type of disease, the severity of the disease, the types and contents of the active ingredients and other ingredients contained in the composition, the type of formulation, the patient's age, body weight, general health status, sex and diet, the time of administration, the route of administration, the secretion rate of the composition, the duration of treatment, and concurrently used drugs. For adults, the pharmaceutical composition for treatment may be administered into the body in an amount of 50 ml to 500 ml at a time, the compound may be administered at a dose of 0.1 ng/kg to 10 mg/kg, and a monoclonal antibody may be administered at a dose of 0.1 ng/kg to 10 mg/kg. Administration may be performed once to 12 times a day, and when administration is performed 12 times a day, administration may be performed once every 2 hours. In addition, the pharmaceutical composition of the present invention may be administered alone or in combination with other therapies known in the art, such as chemotherapy, radiotherapy, and surgery, for the treatment of the cancer of interest. The pharmaceutical composition of the present invention may also be administered in combination with other treatments designed to enhance immune responses, e.g., by co-administration with adjuvants or cytokines (or nucleic acids encoding cytokines), as is well known in the art. Other standard delivery methods, e.g., biolistic transfer or *ex vivo* treatment, may also be used. In *ex vivo* treatment, antigen presenting cells (APCs), dendritic cells, peripheral blood mononuclear cells, or bone marrow cells may be obtained from a patient or an appropriate donor and activated *ex vivo* with the pharmaceutical composition of the present invention, and then administered into the patient.

In the present specification, the term "therapeutically effective amount" refers to an amount of the composition containing a pharmacological ingredient sufficient to provide a therapeutic or prophylactic effect to a subject to whom the pharmaceutical composition of the present invention is to be administered. Accordingly, the term "therapeutically effective amount" is meant to include a "prophylactically effective amount".

According to a specific embodiment of the present invention, the composition is co-administered.

According to a specific embodiment of the present invention, the *Salmonella* strain is selected from the group consisting of *Salmonella typhimurium, Salmonella choleraesuis, Salmonella enteritidis, Salmonella dublin, Salmonella. typhisuis, Salmonella derby,* and *Salmonella gallinarum.* Specifically, the *Salmonella* strain may be *Salmonella typhimurium,* without being limited thereto.

In the present invention, the term "*Salmonella enterica*" refers to Enterobacteriaceae that inhabit the intestinal tracts of primates including humans, and secrete cytolysin known as an exotoxin. Cytolysin that is secreted in this way is a cytotoxic protein having a molecular weight of about 34 kDa, and is known to cause hemolysis (the destruction of red blood cells) in the intestines of primates including humans and to form pores in the membrane of normal cells to induce cell lysis, which leads to death due to severe vascular inflammation and the necrosis of local tissue. However, recent research results indicate that the cytolysin isolated and purified from *Salmonella enterica* acts specifically on the cancer tissue in the intestinal tract in the body, thereby inducing the death of the cancer tissue. Thus, the cytolysin has attracted attention as a next-generation anticancer therapeutic agent. Therefore, bacteria transformed with a gene secreting the cytoxic substance cytolysin may have a very high potential for use as an anticancer therapeutic agent for targeting cancer tissue.

According to the present invention, host cells may include cells of mammalian, plant, insect, fungal or cellular origin. For example, the host cell may be, but is not limited to, at least one type selected from the group consisting of bacterial cells such as *Escherichia coli*, *Streptomyces* or *Salmonella* sp. strains; fungal cells such as yeast cells or *Pichia pastoris;* insect cells such as Drosophila or Spodoptera Sf9 cells; animal cells such as Chinese hamster ovary (CHO) cells, SP2/0 (mouse myeloma), human lymphoblastoid, COS, NSO (mouse myeloma), 293T cells, bow melanoma cells, HT-1080 cells, baby hamster kidney (BHK) cells, human embryonic kidney (HEK) cells, or PERC.6 cells (human retinal cells; and plant cells. For the purposes of the present invention, the strain may be at least one selected from the group consisting of anaerobic strains, for example, *Salmonella* sp. strains, *Clostridium* sp. strains, *Bifidobacterium* sp. strains, and *E. coli* strains. Preferably, the strain may be at least one selected from the group consisting of *Salmonella typhimurium*, *Salmonella choleraesuis*, and *Salmonella enteritidis.* More preferably, the strain may be *Salmonella typhimurium*, without being limited thereto.

The strain of the present disclosure may be an attenuated strain.

The term "attenuated" as used in the present invention means modifying a gene or the like so as to reduce toxicity and other side effects, which may occur when a microorganism is administered to a patient. For the purposes of the present disclosure, when the strain is a *Salmonella* sp. strain, at least one gene selected from the group consisting of *aroA, aroC, arod, aroE, Rpur, htrA, ompR, ompF, ompC, galE, cya, crp, cyp, phoP, phoQ, rfaY, dksA, hupA, sipC, clpB, clpP, clpX, pab, nadA, pncB, pmi, rpsL, hemA, rfc, poxA, galU, cdt, pur, ssa, guaA, guaB, flid, flgK, flgL, relA* and *spoA* may be modified for attenuation, without being limited thereto. The method for modifying the gene of the present invention may be performed by a method of deleting or disrupting various genes as known in the art. For example, the deletion or disruption method may be performed by a method such as homologous recombination, chemical mutagenesis, irradiation mutagenesis, or transposon mutagenesis.

In the present invention, the strain targets the inside of cancer tissue, which is an oxygen-deficient environment which is very suitable for the growth of an anaerobic strain and shows incomplete blood vessel formation, and thus when a recombinant vector comprising a reporter protein that may be imaged in real time and an anticancer protein is introduced into this strain so that the expression levels of the reporter protein and the anticancer protein can be balanced, it is possible to very effectively diagnose and treat cancer.

According to a specific embodiment of the present invention, the immune checkpoint inhibitor is selected from the group consisting of cytotoxic T-lymphocyte-associated antigen-4 (CTLA-4), programed cell death protein 1 (PD-1), programmed death-ligand 1 (PD-L1), KIR, LAG3, CD137, OX40, CD47, CD276, CD27, and GITR.

Specifically, the immune checkpoint inhibitor is programmed death-ligand 1 (PD-L1).

In the present invention, the term "programmed death-ligand 1 (PD-L1)" refers to a protein on the surface of cancer cells or hematopoietic cells. When PD-L1 and PD-L2, which are also called CD274 and B7-H1 and are proteins on the surface of cancer cells, bind to PD-1, a protein on the surface of T cells, the T cells cannot attack cancer cells. Anticancer immunotherapeutic agents bind to the PD-1 receptor of T cells and inhibit the evasion function of cancer cells.

According to a specific embodiment of the present invention, the cancer is selected from the group consisting of melanoma, fallopian tube cancer, brain cancer, small intestine cancer, esophagus cancer, lymph node cancer, gallbladder cancer, blood cancer, thyroid cancer, endocrine cancer, oral cancer, liver cancer, biliary tract cancer, colorectal cancer, rectal cancer, cervical cancer, ovarian cancer, kidney cancer, gastric cancer, duodenum cancer, prostate cancer, breast cancer, brain tumor, lung cancer, undifferentiated thyroid cancer, uterine cancer, colon cancer, bladder cancer, ureteral cancer, pancreatic cancer, bone/soft tissue sarcoma, skin cancer, non-Hodgkin lymphoma, Hodgkin lymphoma, multiple myeloma, leukemia, myelodysplastic syndrome, acute lymphoblastic leukemia, acute myeloid leukemia, chronic lymphocytic leukemia, chronic myeloid leukemia, and solitary myeloma.

Since cancer has already been described in detail in the present invention, the description thereof will be omitted to avoid excessive duplication.

According to a specific embodiment of the present invention, the composition inhibits cancer growth or cancer metastasis.

In the present specification, the term "cancer metastasis" refers to the spread of cancer cells from the primary organ or site to other distal sites. Metastatic cancer is cancer having the ability to metastasize or cancer that has metastasized. In particular, the metastatic cancer may metastasize to the liver, lungs, bones, lymph nodes, or abdominal cavity, without being limited thereto. The metastatic cancer is difficult to treat, and its progression and treatment thereof may be more complicated than that in the initial treatment process.

In the present invention, "cancer recurrence" means that cancer is not detectable after treatment and then is detected again after a certain period of time. Recurrent cancer refers to cancer resulting from cancer recurrence as described above. When cancer recurs, it is often difficult to resect, and even when it can be resected, significant surgery may be necessary. Additionally, there may be limitations in chemotherapy and radiotherapy.

According to a specific embodiment of the present invention, the *Salmonella* may be *Salmonella* transformed with a vector containing a DNA construct comprising: a gene encoding a regulatory protein; the promoter of the gene encoding the regulatory protein; a first promoter and a second promoter, which are induced by the regulatory protein; and any one selected from the group consisting of a gene encoding an anticancer protein downstream of the first promoter and the second promoter, a gene encoding a cytokine, a gene encoding a chemokine, a gene encoding an immune modulator, a cancer antigen-specific oligonucleotide, and a gene encoding a reporter protein.

In the present specification, the term "cytokine" refers to proteins secreted by immune cells, and the cytokine of the present invention may include any cytokine that may be used in cancer immunotherapy capable of inducing the death of disease-related cells, for example, cancer cells, by regulating host immune response. Preferably, examples of the cytokine include, but are not limited to, IFN-α2, IL-2, IL-15, IL-21 and IL-12.

In the present specification, the term "chemokine" refers to one functioning to control the migration of cells between tissues and the positioning and interactions of cells within tissues. The chemokine may include any chemokine that may mediate the host response to diseases, for example, cancer, by directing the trafficking of leukocytes into the tumor microenvironment. Preferably, examples of the chemokine include, but are not limited to, CXCR3, CCR5, etc.

In the present specification, the term "immune modulator" refers to a modulator which enables various treatments by utilizing the intrinsic immune system of an individual, and may include any modulator that can induce the death of disease-related cells, for example, cancer cells, by activating immune cells.

In the present specification, the "anticancer protein" refers to a peptide having a function capable of directly or indirectly inducing the death of cancer cells. The anticancer protein may be, for example, at least one selected from the group consisting of a toxin protein, an antibody specific for a cancer antigen or a fragment of the antibody, a tumor suppressor protein, an angiogenesis inhibitor, a cancer antigen, a prodrug-converting enzyme, a pro-apoptotic protein, and flagellin, without being limited thereto.

Since the first promoter and second promoter of the present invention are simultaneously inducible by a single regulatory protein expressed by a separate promoter, the expression levels of proteins encoded by the genes, operably linked downstream the first promoter and the second promoter, in a host strain or cell, may be balanced with each other, unlike the case in which the gene encoding the regulatory protein gene is operably linked downstream of the second promoter. As such, when the DNA construct is used, diagnosis and treatment may be performed simultaneously.

According to the present invention, the term "DNA construct" refers to a construct that enables expression of a desired protein or the like when introduced into a host strain or cell by transformation, and comprises not only a gene encoding the desired protein, but also nucleotide sequences corresponding to promoters, which are essential regulatory elements operably linked so that the gene can be expressed.

The term "promoter" as used in the present invention refers to a nucleotide sequence which is present upstream of an operably linked gene in a host strain or cell, and is the nucleotide sequence of a specific region of the DNA construct to which RNA polymerase may bind in order to initiate transcription.

According to a specific embodiment of the present invention, the regulatory protein is a TetR protein, the promoter of the gene encoding the regulatory protein is an OXB 1 promoter, the first promoter induced by the regulatory protein is a tetA promoter, and the second promoter derived by the regulatory protein is a tetR promoter.

According to a specific embodiment of the present invention, expression of the regulatory protein is regulated by a cis-acting element or a trans-acting element.

Regulation of expression of the regulatory protein of the present invention may be achieved by a cis-acting element (cis-regulatory element; CRE) or a trans-acting element (trans-regulatory element; TRE).

In the present invention, the term "regulation" or "regulation of expression" may mean that transcription and translation of a specific gene are activated or inhibited.

According to a specific embodiment of the present invention, the cis-acting factor may be at least one selected from the group consisting of a ribosome binding site (RBS), a 5'-untransrated region (5'-UTR), a transcription factor binding site, and terminators, without being limited thereto.

In the present specification, the term "ribosome binding site (RBS)" is also referred to as the Shine-Dalgarno sequence (SD sequence). After the genetic information contained in DNA is transcribed into messenger RNA (mRNA), a ribosome must bind to this mRNA in order for translation to occur. At this time, the ribosome binding site means a short sequence that is present on the mRNA so that the ribosome can bind effectively to the mRNA.

In the present specification, the term "5'-untranslated region (5'-UTR)" refers to untranslated regions flanking both sides of a 5' coding region which is translated into amino acids of mRNA. It is considered a junk in the evolutionary process, but is known to play a major role in regulating gene expression.

In the present specification, the term "transcription factor binding site" refers to a DNA region that serves to turn on or off a specific gene nearby. The transcription factor binding site may be at least one selected from the group consisting of a promoter of the gene encoding the regulatory protein, an enhancer, and a silencer, without being limited thereto.

According to a specific embodiment of the present invention, the transcription factor binding site is selected from the group consisting of a promoter of the gene encoding the regulatory protein, an enhancer, and a silencer.

The enhancer of the present invention is a sequence found in both prokaryotes and eukaryotes, and generally has a 50 to 1,500 bp region, is located upstream or downstream from the starting point of the nearby gene, and induces binding of the transcription factor.

The silencer of the present invention has the same mechanism as the enhancer and antagonizes the enhancer effect. The transcription factor that binds to the silencer is a repressor. The enhancer and the silencer may be present in regions adjacent to each other, or may be present in the same region in which transcription factors thereof are different.

According to a specific embodiment of the present invention, the promoter of the gene encoding the regulatory protein is a weak promoter.

The promoter of the gene encoding the regulatory protein of the present invention may include any promoter whose activity can be induced in the environmental conditions and developmental conditions of most host strains or cells. Preferably, the promoter may be a weak promoter.

In the present specification, the term "weak promoter" refers to a promoter that induces a transcript, transcribed from the gene operably linked downstream thereof, to be expressed at a level of 1 × 10⁻² or less, preferably 1 × 10⁻³ or less. The weak promoter may include any promoter that allows the transcript to be expressed at a level of 1 × 10⁻³ or less as described above. For example, the weak promoter may be at least one selected from the group consisting of an *E. coli* σ70 promoter, an *E. coli* σS promoter, an *E. coli* σ32 promoter, a *B. subtilis* σA promoter, a *B. subtilis* σB promoter, the *Salmonella*-derived promoter K112706 or K112707, a bacteriophage T7 promoter, a bacteriophage SP6 promoter, a yeast-derived promoter, the eukaryotic promoter I712004 or K076017, an OXB1 promoter, and a plant-derived promoter, without being limited thereto.

The *E. coli* σ70 promoter of the present invention may be at least one selected from the group consisting of I14018, I14033, I14034, I732021, I742126, J01006, J23103, J23109, J23112, J23113, J23117, J23119, J23150, J23151, J44002, J48104, J56015, J64951, K088007, K119000, K119001, K1330002, K137029, K137030, K137031, K137032, K137085, K137086, K137087, K137088, K137089, K137090, K137091, K1585100, K1585101, K1585102, K1585103, K1585104, K1585105, K1585106, K1585110, K1585113, K1585115, K1585116, K1585117, K1585118, K1585119, K2486171, K256002, K256018, K256020, K256033, K292000, K823007, K823010, K823013, M13101, M13102, M13103, M13104, M13105, M13106, M13108, M13110, M31519, R1074, R1075, and S03331, without being limited thereto.

The *E. coli* σS promoter of the present invention may be J45992 or J45993, without being limited thereto.

The *E. coli* σ32 promoter of the present invention may be J45504, K1895002 or K1895003, without being limited thereto.

The *B. subtilis* σA promoter of the present invention may be at least one selected from the group consisting of K143012, K143013, K823000, K823002 and K823003, without being limited thereto.

The *B. subtilis* σB promoter of the present invention may be K143010, K143011 or K143013, without being limited thereto.

The bacteriophage T7 promoter of the present invention may be at least one selected from the group consisting of I719005, J34814, J64997, K113010, K113011, K113012, K1614000, R0085, R0180, R0181, R0182, R0183, Z0251, Z0252 and Z0253, without being limited thereto.

The bacteriophage SP6 promoter of the present invention may be J64998, without being limited thereto.

The yeast-derived promoter of the present disclosure may be at least one selected from the group consisting of I766557, J63005, K105027, K105028, K105029, K105030, K105031, K122000, K124000, K124002, K319005, M31201, K2365040, K2365036, K2365041, K2365042, K2365032, K2365051, K2365514, K2365515 and K2365516, without being limited thereto.

The plant-derived promoter of the present disclosure may be at least one selected from the group consisting of PLPR0203, PLPR0210, PLPR0177, PLPR0193, PLPR0507, PLPR0422, PLPR0228, PLPR0226, PLPR0223, PLPR0040, PLPR0465, PLPR0232, PLPR0205, PLPR0247, PLPR0328, PLPR0525, AtREG383, AtREG415, AtREG416, OsREG438, OsREG443, OsREG501, PpREG186, PpREG194 and PpREG197, without being limited thereto.

In the present invention, the weak promoter may be the OXB1 promoter represented by SEQ ID NO: 4, without being limited thereto.

For the purposes of the present invention, when the gene encoding the regulatory protein is operably linked downstream of the weak promoter, it is possible to control transcription such that transcription of the gene present downstream of the first and second promoters may occur specifically only when a substance that inhibits the regulatory protein is administered, unlike the case where the gene is operably linked downstream of the first promoter or the second promoter.

The promoter of the gene encoding the regulatory protein of the present invention may have the nucleotide sequence of SEQ ID NO: 2 corresponding to the -35 site from the gene encoding the regulatory protein, and the nucleotide sequence of SEQ ID NO: 3 corresponding to the -10 site, without being limited thereto.

The weak promoter may induce a transcript, transcribed from a gene operably linked downstream of the promoter, to be expressed at a level of 1 × 10⁻² or less.

According to a specific embodiment of the present invention, the trans-acting element may be at least one selected from the group consisting of a transcription factor, an aptamer, sRNA, and antisense RNA (asRNA), without being limited thereto.

In the present invention, the transcription factor is a protein that helps to turn on or off a specific gene by binding to the transcription factor binding site.

In the present specification, the term "aptamer" is a part of a riboswitch, and collectively refers to oligonucleotide or peptide substances capable of binding to a specific target molecule. The aptamer may be a peptide aptamer or a nucleic acid aptamer. The riboswitch is a kind of mRNA that regulates the expression of genes, and examples thereof include, but are not limited to, a glmS riboswitch, an FMN riboswitch, a Cobalamin riboswitch, and the like.

In the present invention, the sRNA or the antisense RNA (asRNA) refers to a single-stranded RNA capable of complementarily binding to a specific RNA. The antisense RNA binds complementarily to sense RNA, which is a messenger RNA (mRNA) expressing a specific protein, thereby regulating the expression of the protein.

According to a specific embodiment of the present invention, the anticancer protein is selected from the group consisting of a toxin protein, an antibody specific for a cancer antigen or a fragment of the antibody, a tumor suppressor protein, an angiogenesis inhibitor, a cancer antigen, a prodrug-converting enzyme, and a pro-apoptotic protein.

In the present specification, the term "tumor suppressor protein" refers to a protein which maintains its function in normal cells, but causes normal cells to indiscriminately divide and grow into cancer cells when the function thereof is lost. Examples of the tumor suppressor protein include, but are not limited to, retinoblastoma (RB) protein, p53 protein, adenomatous polyposis coli (APC) protein, phosphatase and tensin homologue (PTEN) protein, cyclin dependent kinase inhibitor 2A (CDKN2A) protein, and the like.

In the present invention, the antibody specific for cancer antigen and the fragment of the antibody is an antibody capable of specifically binding to an antigen which is a protein expressed at a high level specifically on the surface or cytoplasm of a cancer cell. For example, the antibody or the fragment thereof may be an antibody specific for HER2 that is expressed at a high level specifically in breast cancer or gastric cancer cells, without being limited thereto.

The term "antibody" as used in the present disclosure refers to a protein molecule capable of binding specifically to an antigenic site of a protein or peptide molecule. The type of the antibody is not particularly limited, and examples thereof include polyclonal antibodies, monoclonal antibodies, or antibody fragments having an antigen-binding property, and include all types of immunoglobulin antibodies. In addition, examples of the antibody include specific antibodies such as humanized antibodies. Examples of the antibody include not only a whole antibody having two full-length light chains and two full-length heavy chains, but also a functional fragment of an antibody molecule. The "functional fragment of an antibody molecule" refers to a fragment having at least an antigen-binding function, and examples thereof include, but are not limited, Fab, F(ab'), F(ab')2, Fv, etc.

The "antibody" of the present invention may be produced by a conventional method after cloning the gene encoding the cancer antigen of the present invention into an expression vector according to a conventional method to obtain a protein encoded by the gene.

In the present specification, the term "angiogenesis inhibitor" refers to a protein or compound having a function capable of directly or indirectly inducing the death of cancer cells by inhibiting the formation of new blood vessels around cancer cells. Preferably, examples of the angiogenesis inhibitor include, but are not limited to, angiostatin, endostatin, thrombospondin, and protease inhibitory proteins.

In the present specification, the term "cancer antigen" refers to a protein antigen which is expressed in cancer cells but is rarely expressed in normal cells, thereby inducing an anti-tumor immune response, thus inducing the direct or indirect death of cancer cells. Preferably, the cancer antigen of the present invention may be α-fetoprotein (AFP), vascular endothelial growth factor receptor 2 (VEGFR2), Survivin, Legumain, prostate cancer-specific antigen (PCSA), or the like, without being limited thereto.

According to a specific embodiment of the present invention, the toxin protein is selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA).

In the present specification, the term "toxin protein" refers to a protein having a function capable of directly or indirectly inducing the death of cancer cells. For example, the toxin protein may be at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA). More preferably, the toxin protein may be cytolysin A consisting of the amino acid sequence represented by SEQ ID NO: 1, without being limited thereto.

According to a specific embodiment of the present invention, the tumor suppressor protein is selected from the group consisting of retinoblastoma (RB) protein, p53 protein, adenomatous polyposis coli (APC) protein, phosphatase and tensin homologue (PTEN) protein, and cyclin dependent kinase inhibitor 2A (CDKN2A) protein.

Since the tumor suppressor protein has already been described in detail in the present invention, the description thereof will be omitted to avoid excessive overlapping.

According to a specific embodiment of the present invention, the angiogenesis inhibitor is selected from the group consisting of angiostatin, endostatin, thrombospondin, and protease inhibitory proteins.

Since the angiogenesis inhibitor has already been described in detail in the present invention, the description thereof will be omitted to avoid excessive overlapping.

According to another aspect of the present invention, the present invention provides a method of preventing or treating cancer by administering to a subject a pharmaceutical composition comprising a *Salmonella* strain and an immune checkpoint inhibitor.

According to still another aspect of the present invention, provided is the use of a *Salmonella* strain and an immune checkpoint inhibitor for preventing or treating cancer.

### Advantageous Effects

The features and advantages of the present invention are summarized as follows:
(a) The present invention provides a pharmaceutical composition for preventing or treating cancer comprising a *Salmonella* strain and an immune checkpoint inhibitor as active ingredients.
(b) The composition of the present invention may be useful as a prophylactic or therapeutic composition of improving the survival rate by significantly reducing the tumor size by co-administering bacteria and an immune checkpoint inhibitor in cancer, especially a type of cancer that is resistant and difficult to treat by a single anticancer therapy.

### Brief Description of Drawings

FIG. 1a shows an experimental schedule for co-administration of *Salmonella* and an immune checkpoint inhibitor according to one embodiment of the present invention.
FIG. 1b is a graph showing the anticancer effect by the change in tumor volume upon co-administration of *Salmonella* and an immune checkpoint inhibitor according to an experimental example of the present invention.
FIG. 1c shows results indicating the survival rate upon co-administration of *Salmonella* and an immune checkpoint inhibitor according to an experimental example of the present invention.
FIG. 1d shows results indicating the change in tumor volume after reimplanting the tumor into mice from which the tumor has been removed by combination therapy, according to an experimental example of the present invention.
FIG. 2a shows an experimental schedule for reimplanting a tumor after CT26 cell tumor implantation according to an experimental example of the present invention.
FIG. 2b is a graph showing the change in tumor volume after reimplanting the tumor into mice from which the tumor has been removed by combination therapy, according to an experimental example of the present invention.
FIG. 3a is a graph showing the change in tumor volume following injecting each of IgG, anti-PD-L1, CNC018+IgG and CNC018+anti-PD-L1 after MC38 cell tumor implantation according to an experimental example of the present invention.
FIG. 3b is a graph showing the survival rate following injecting each of IgG, anti-PD-L1, CNC018+IgG and CNC018+anti-PD-L1 after MC38 cell tumor implantation according to an experimental example of the present invention.
FIG. 4a is a graph showing the change in tumor volume following injecting each of IgG, anti-PD-L1, SL△ppGpp+IgG and SL△ppGpp+anti-PD-L1 after MC38 cell tumor implantation according to an experimental example of the present invention.
FIG. 4b is a graph showing the survival rate following injecting each of IgG, anti-PD-L1, SL△ppGpp+IgG and SL△ppGpp+anti-PD-L1 after MC38 cell tumor implantation according to an experimental example of the present invention.
FIG. 5a shows an experimental schedule for reimplanting a tumor after MC38 cell tumor implantation according to an experimental example of the present invention.
FIG. 5b is a graph showing the change in tumor volume following reimplanting a tumor into CNC018- and SL△ppGpp-pretreated mice after MC38 cell tumor implantation according to an experimental example of the present invention.
FIG. 6a shows results indicating the change in mouse spleen size following combination therapy after tumor implantation according to an experimental example of the present invention.
FIG. 6b shows results indicating the change in mouse spleen weight following combination therapy after tumor implantation according to an experimental example of the present invention.
FIG. 7a shows results indicating the activation of memory CD8+ T cells by combination therapy after tumor implantation according to an experimental example of the present invention.
FIG. 7b shows results indicating the activation of memory CD4+ memory T cells by combination therapy after tumor implantation according to an experimental example of the present invention.
FIG. 8a shows results indicating the activation of IFN-γ-specific CD4+ T cells by combination therapy according to an experimental example of the present invention.
FIG. 8b shows results indicating the activation of IFN-γ-specific CD8+ T cells by combination therapy according to an experimental example of the present invention.
FIG. 9a shows the results of evaluating the inhibitory effect of combination against cancer metastasis to the lung according to an experimental example of the present invention.
FIG. 9b is a graph showing the results of evaluating the inhibitory effect of combination against cancer metastasis to the lung according to an experimental example of the present invention.
FIG. 10a shows the results of evaluating the inhibitory effect of combination against cancer metastasis to the liver in mice according to an experimental example of the present invention.
FIG. 10b is a graph showing the results of evaluating the inhibitory effect of combination against cancer metastasis to the liver in mice according to an experimental example of the present invention.

### Best Mode

The present invention is directed to a composition for the highly reliable prevention and treatment of a cancer that is resistant and difficult to treat by a single anticancer therapy and for which there is no effective therapeutic method. The present inventors have developed a composition for preventing or treating cancer using a *Salmonella* strain and an immune checkpoint inhibitor as active ingredients, and have found that tumor growth is significantly inhibited and reduced by the combination therapy of a *Salmonella* strain that selectively acts on cancer and an immune checkpoint inhibitor including PD-L1. Thus, the present invention may provide a fundamental and efficient therapeutic method and composition for cancer.

### Mode for Invention

Hereinafter, the present invention will be described in more detail by way of examples. It will be obvious to those skilled in the art that these examples are only for explaining the present disclosure in more detail, and the scope of the present invention according to the subject matter of the present invention is not limited by these examples.

### Examples

### [Preparation Example 1] Cancer Cell Lines and Culture Conditions

The CT26 colon cancer cell lines CRL-2638 and HB-8064 (ATCC, USA) and the murine colorectal adenocarcinoma cell line MC38 (Massachusetts General Hospital and Harvard Medical School, USA and Chonnam National University, Korea) were used in the experiment.

Using high-glucose DMEM (Dulbecco's Modified Eagles Medium) (Catalog No. #LM 001-05, Welgene, Korea) containing 10% fetal bovine serum (FBS) and 1% penicillin-streptomycin, the cells were cultured in a 5% CO₂ incubator at 37°C.

### [Preparation Example 2] Preparation of Salmonella Strains Having Plasmids Introduced Thereinto

As *Salmonella* strains, SL△ppGpp (ΔrelA, ΔspoT) and CNC018 (ΔrelA, ΔspoT, ΔSPI1, ΔSPI2), which are ppGpp-deficient *Salmonella typhimurium (S. typhimurium)*, were used.

Each constructed plasmid was transformed into the *Salmonella* strain by electroporation, and each of the transformed strains was cultured overnight in an LB containing 100 µg/ml ampicillin. Thereafter, each of the cultures was diluted at a ratio of 1: 100 with a fresh LB medium containing ampicillin and cultured in a shaking incubator under conditions of 200 rpm and 37°C. Each of the cultures was centrifuged, and the strain pellet was collected, washed using a PBS buffer, and then used in the experiment.

### [Preparation Example 3] Preparation of Experimental Animal Models

5 to 6-week-old C57BL/6 and BALB/C mice (Orient Company, Korea) weighing 20 to 30 g were used. MC38 or CT26 of Preparation Example 1 was subcutaneously injected into the flank of each of the mice, thereby preparing tumor animal models.

For imaging of the tumor animal model and evaluation of the tumor volume, 2% isoflurane was used for anesthesia, and 200 mg/kg of ketamine and 10 mg/kg of xylasine were used during surgery.

The tumor volume (mm³) was calculated using the equation "(length × height × width)/2", and when the tumor volume in the animal model reached 1,500 mm³ or larger, the animal model was euthanized.

### [Experimental Example 1] Evaluation (1) of Anticancer Effect of Co-Administration of Salmonella Strain and Immune Checkpoint Inhibitor

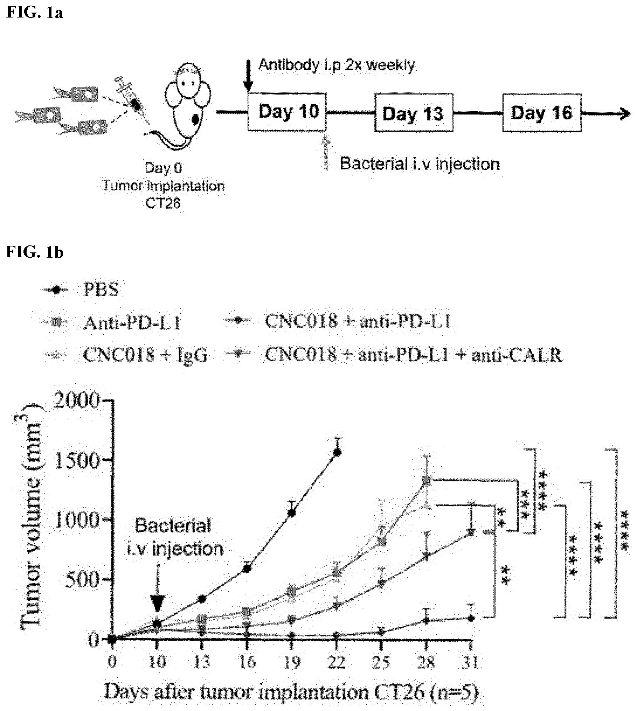

In order to evaluate the anticancer effect of co-administration of the *Salmonella* strain and the immune checkpoint inhibitor anti-PD-L1, an *in vivo* experiment was performed in the same manner as shown in FIG. 1a. After each drug was injected into the CT26 tumor mouse model, the strain was injected intravenously on day 10.

After injection of each ofPBS, anti-PD-L1, CNC018-IgG, CNC018-anti-PD-L1-anti-CALR, and CNC018-anti-PD-L1, changes in the tumor volume were examined (FIG. 1b).

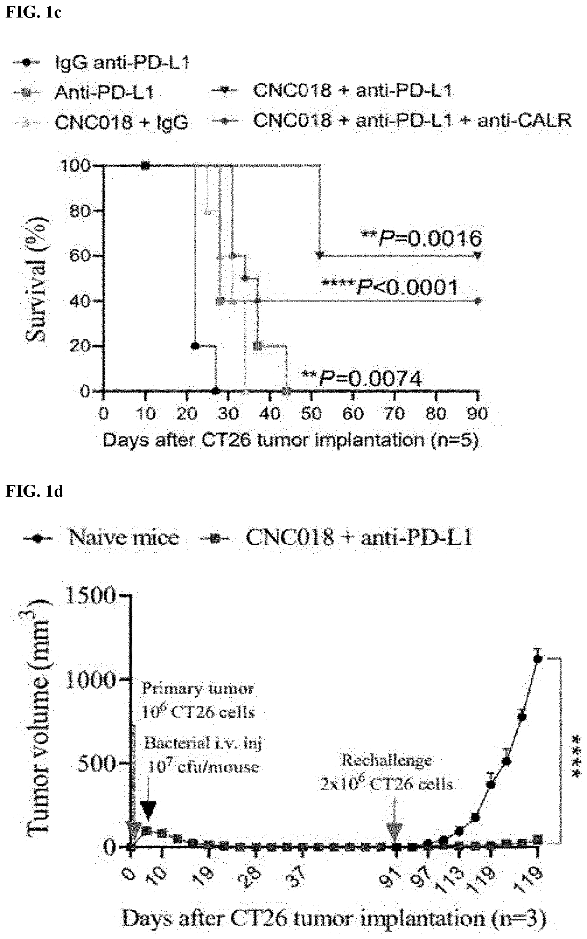

When the attenuated Salmonella strain and anti-PD-L1 were co-administered, the tumor size was significantly reduced compared to that in the control group (PBS), and the survival rate of mice was also greatly improved (FIG. 1c). In addition, as shown in FIG. 1d, as a result of evaluating changes in the tumor volume after reimplanting the tumor on day 91 into mice from which the tumor has been removed by combination therapy, it was confirmed that the change in tumor volume was very insignificant compared to the control group (naive mouse), suggesting that CNC018-anti-PD-L1 was also effective against tumor recurrence.

### [Experimental Example 2] Evaluation (2) of Anticancer Effect of Co-Administration of Salmonella Strain and Immune Checkpoint Inhibitor

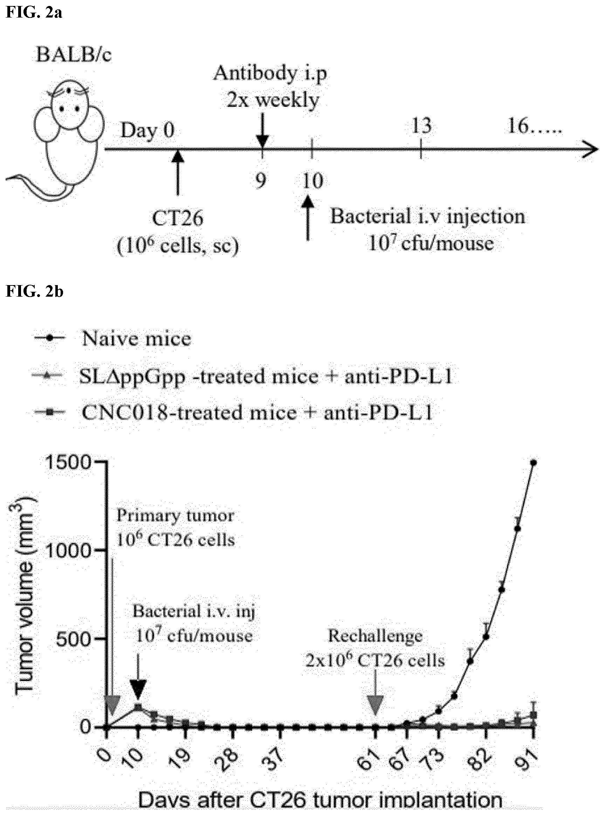

In order to evaluate the anticancer effect of co-administration of the *Salmonella* strain and the immune checkpoint inhibitor anti-PD-L1, in particular, evaluate the inhibitory effect of co-administration against cancer recurrence after reimplanting the tumor into mice from which the tumor has been removed by combination therapy, an *in vivo* experiment was performed in the same manner as shown in FIG. 2a, and the growth inhibitory effect of SL△ppGpp-anti-PD-L1 or CNC018-anti-PD-L1 in the tumor against the CT26 cell line was evaluated. In the experiment, 1×10⁷ CFU of the attenuated *Salmonella* strain was intravenously administered to the CT26 mouse model, but not administered to the control group. The resulting tumor volumes are shown in FIG. 2b.

As shown in FIG. 2b, it was confirmed that, when the mice were pretreated with each of SL△ppGpp-anti-PD-L1 and CNC018-anti-PD-L1, tumor inhibition in the mice was significantly increased compared to that in the control group, suggesting that SL△ppGpp-anti-PD-L1 and CNC018-anti-PD-L1 have anticancer and recurrence inhibitory effects.

### [Experimental Example 3] Evaluation (3) of Anticancer Effect of Co-Administration of Salmonella Strain and Immune Checkpoint Inhibitor

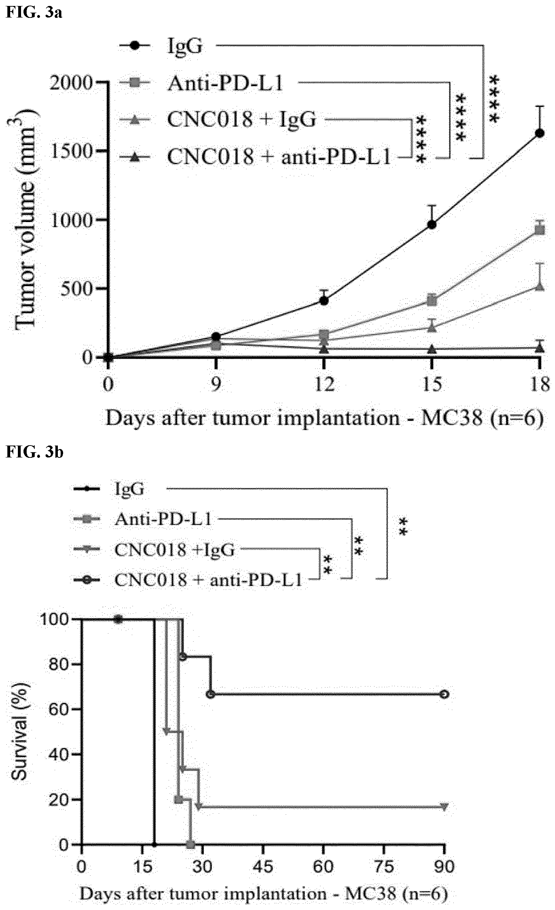

To evaluate the anticancer effect of co-administration of the *Salmonella* strain and the immune checkpoint inhibitor anti-PD-L1, an *in vivo* experiment was conducted, and the tumor volume and mouse survival rate after injection of each of IgG, anti-PD-L1, CNC018-IgG, and CNC018-anti-PD-L1 into the MC38 tumor were examined. As a result, it was confirmed that tumor inhibition in the mice co-administered CNC018-anti-PD-L1 was significantly higher than that in the control IgG group, and thus the survival rate of the mice also significantly increased (FIGS. 3a and 3b).

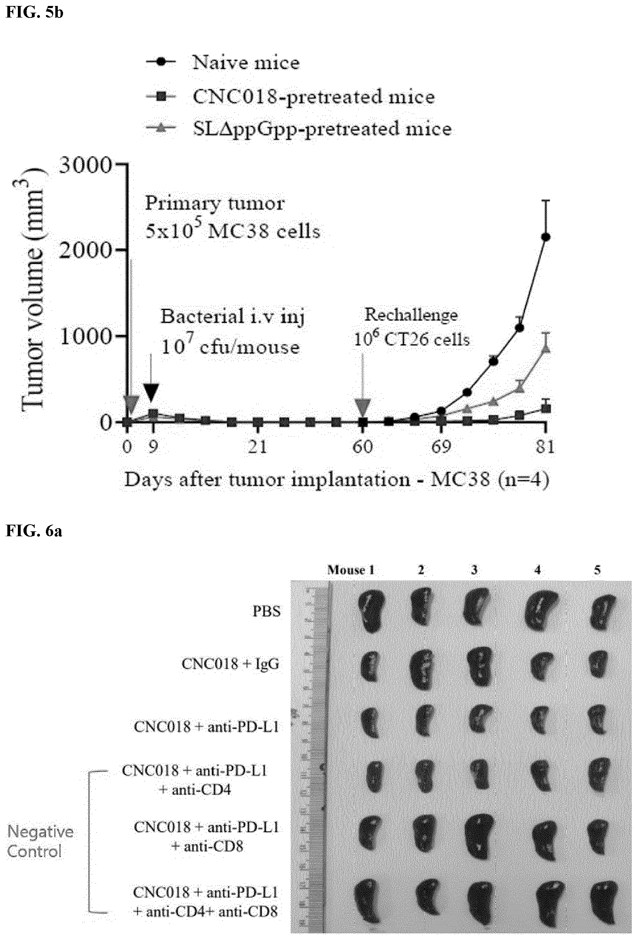

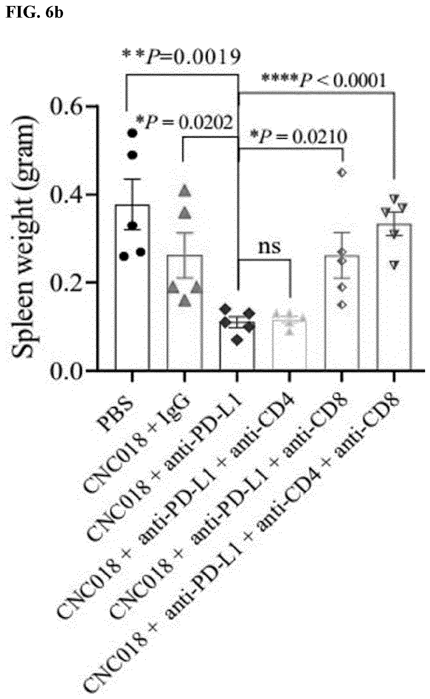

In FIG. 3a, 6 animals per group were used in the experiment, and two-way ANOVA using Tukey's multiple comparisons test was used (****P<0.0001). In FIG. 3b, 6 mice per group were used in the experiment, and Kaplan-Meier survival curves for MC38-tumor-bearing mice were used (**P=0.0016 (CNC018 + anti-PD-L1 vs IgG or CNC018 + IgG; **P=0.0033, CNC018 + anti-PD-L1 vs anti-PD-L1), log-rank (Mantel-Cox test)).

### [Experimental Example 4] Evaluation (4) of Anticancer Effect of Co-Administration of Salmonella Strain and Immune Checkpoint Inhibitor

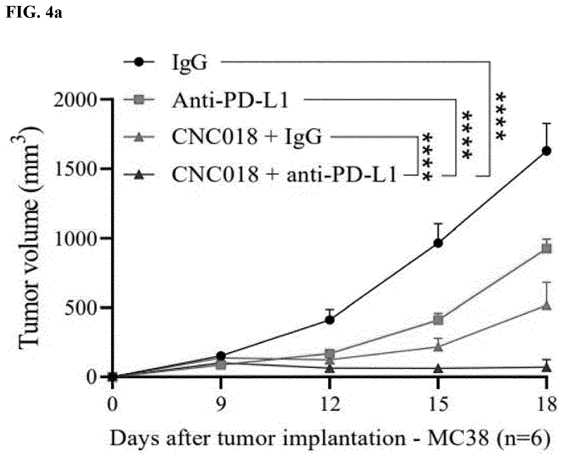

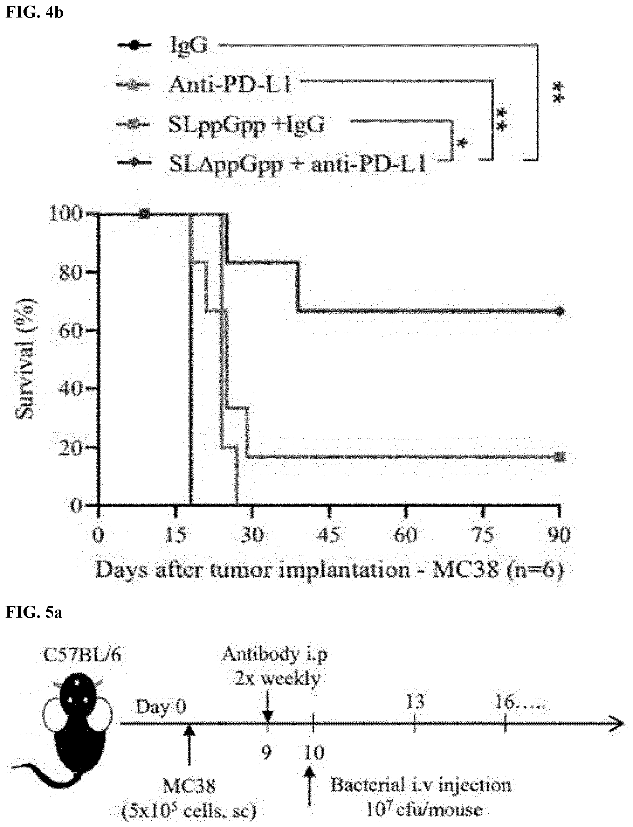

In order to evaluate the anticancer effect of co-administration of the *Salmonella* strain and the immune checkpoint inhibitor anti-PD-L1, an *in vivo* experiment was performed, and the tumor volume and mouse survival rate after injection of each of IgG, anti-PD-L1, SLβ-IgG, and SLβ-anti-PD-L1 into the MC38 tumor were examined. As a result, it was shown that tumor inhibition in the mice co-administered SLβ-anti-PD-L1 was significantly higher than that in the control IgG group, and thus the survival rate of the mice was also very high (FIGS. 4a and 4b).

In FIG. 4a, 6 animals per group were used in the experiment, and two-way ANOVA using Tukey's multiple comparisons test was used (***P=0.0002, ****P<0.0001). In FIG. 4b, 6 mice per group were used in the experiment, and Kaplan-Meier survival curves for MC38-tumor-bearing mice were used (**P=0.0016 (CNC018 + anti-PD-L1 vs IgG or CNC018 + IgG; **P=0.0084, CNC018 + anti-PD-L1 vs anti-PD-L1), *P=0.0432 log-rank (Mantel-Cox test)).

### [Experimental Example 5] Evaluation of Anticancer Effect of Salmonella Strain

In order to evaluate the anticancer effect of the *Salmonella* strain, in particular, evaluate the inhibitory effect of the *Salmonella* strain against cancer recurrence after reimplanting the tumor into mice from which the tumor has been removed, an *in vivo* experiment was performed in the same manner as shown in FIG. 5a, and the growth inhibitory effect of SLβ or CNC018-anti-PD-L1 in the tumor against the MC38 cell line was evaluated. In the experiment, 1×10⁷ CFU of the attenuated *Salmonella* strain was intravenously administered to the MC38 mouse model, but not administered to the control group. The resulting tumor volumes are shown in FIG. 5b.

As shown in FIG. 5b, it was confirmed that, when the mice were pretreated with each of SLβ and CNC018, tumor inhibition in the mice was significantly increased compared to that in the control group (naïve mice), suggesting that SLβ and CNC018-anti-PD-L1 have anticancer and recurrence inhibitory effects.

### [Experimental Example 6] Examination of Spleen Changes after Co-Administration of Salmonella Strain and Immune Checkpoint Inhibitor

To examine changes in the spleen due to co-administration of the *Salmonella* strain and the immune checkpoint inhibitor anti-PD-L1, changes in spleen size and spleen weight of mice after injection of each of PBS, CNC018-IgG, and CNC018-anti-PD-L1 were examined (FIG. 6). As negative controls, CNC018-anti-PD-L1-anti-CD4, CNC018-anti-PD-L1-anti-CD8, and CNC018-anti-PD-L1-anti-CD4-anti-CD8 were used.

As shown in FIG. 6, it was confirmed that, when CNC018-anti-PD-L1 combination therapy was used, it exhibited the smallest changes in spleen size and weight, suggesting that it is stable *in vivo.*

### [Experimental Example 7] Examination (1) of Activation of Tumor-Specific Memory T Cells by Co-Administration of Salmonella Strain and Immune Checkpoint Inhibitor

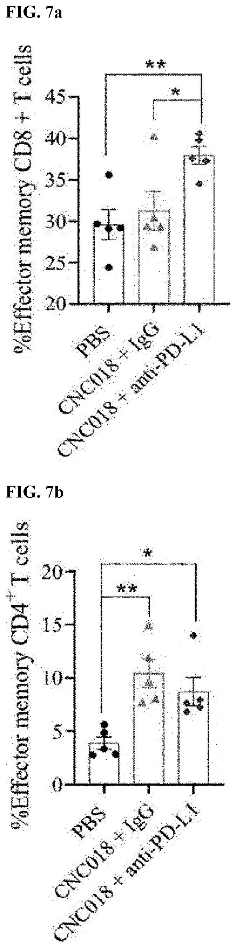

To examine the activation of T cells due to the co-administration of the *Salmonella* strain and the immune checkpoint inhibitor anti-PD-L1, activation of tumor-specific memory T cells was checked after injection of each of PBS, CNC018-IgG, and CNC018-anti-PD-L1. As a result of the experiment, it was shown that both effector memory CD4⁺T cells and effector memory CD8⁺ T cells significantly increased in the co-administered group compared to the control PBS group (FIGS. 7a and 7b).

### [Experimental Example 8] Examination (2) of Activation of Tumor-Specific Memory T Cells by Co-Administration of Salmonella Strain and Immune Checkpoint Inhibitor

To examine the activation of T cells due to the co-administration of the *Salmonella* strain and the immune checkpoint inhibitor anti-PD-L1, activation of tumor-specific memory T cells was checked after injection of each of PBS, CNC018-IgG, and CNC018-anti-PD-L1. 5×10⁵ splenocytes were co-cultured with 2.5×10⁵ irradiated CT26 cancer cells for 12 hours. After stimulation, Golgiplug (1 µl/ml) containing brefeldin A was added to block the intracellular protein transport process for 5 hours. Intracellular staining of interferon-gamma (IFNγ) was performed by FACS analysis.

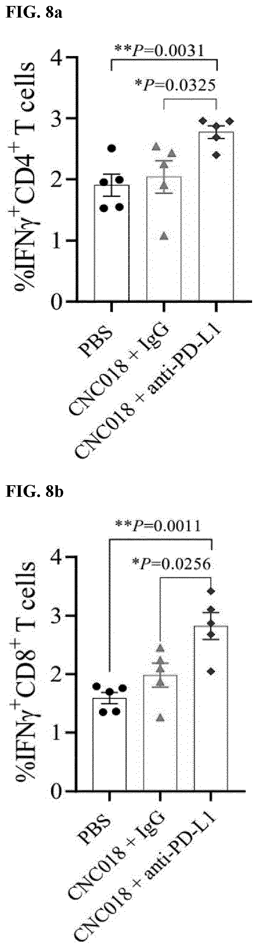

As shown in FIGS. 8a and 8b, both IFNγ⁺CD4⁺ T and IFNγ⁺CD8⁺ T cells significantly increased in the co-administered group compared to the control PBS group.

### [Experimental Example 9] Evaluation (1) of Cancer Metastasis Inhibitory Effect of Co-Administration of Salmonella Strain and Immune Checkpoint Inhibitor

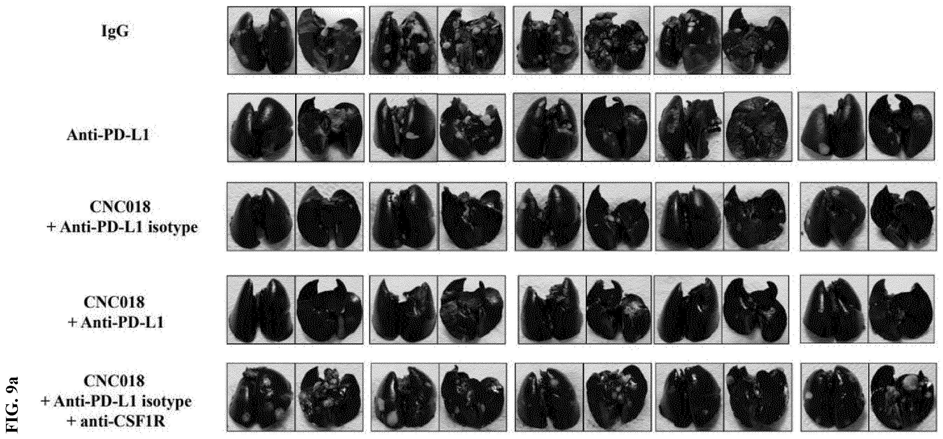

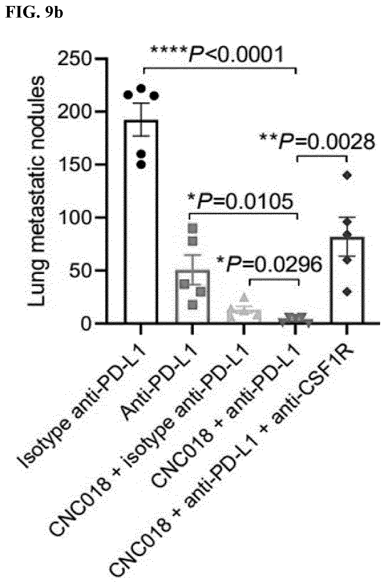

To evaluate the cancer metastasis inhibitory effect of co-administration of the *Salmonella* strain and the immune checkpoint inhibitor anti-PD-L1, IgG, anti-PD-L1, CNC018-anti-PD-L1 isotype, CNC018-anti-PD-L1 and CNC018-anti-PD-L1 isotype-anti-CSFIR were each injected and metastatic nodules in the lung were checked (FIG. 9).

As shown in FIG. 9b, it could be confirmed that the tumor did not metastasize to the lungs in the group co-administered CNC018-anti-PD-L1 compared to the control IgG group, suggesting that the tumor metastasis inhibitory effect of the co-administration was remarkably significant.

### [Experimental Example 10] Evaluation (2) of Cancer Metastasis Inhibitory Effect of Co-Administration of Salmonella Strain and Immune Checkpoint Inhibitor

To evaluate the cancer metastasis inhibitory effect of co-administration of the *Salmonella* strain and the immune checkpoint inhibitor anti-PD-L1, an *in vivo* experiment was conducted to evaluate the growth inhibitory effect of co-administration into the tumor against the HepG2-Luc cell line.

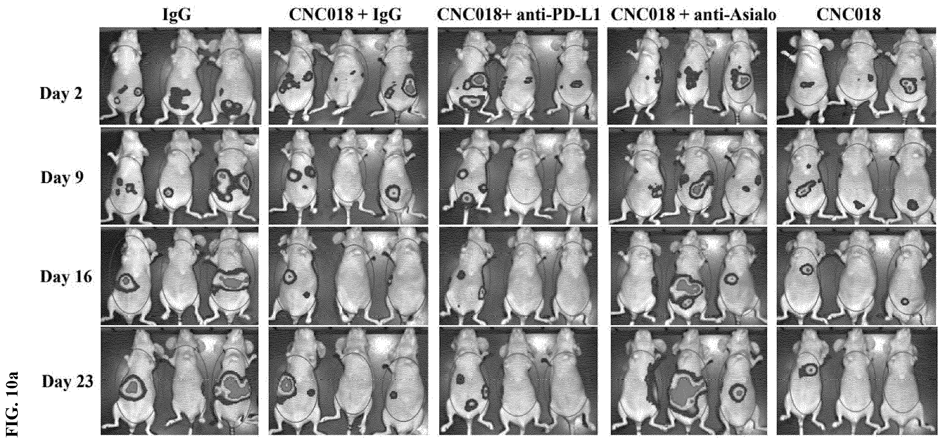

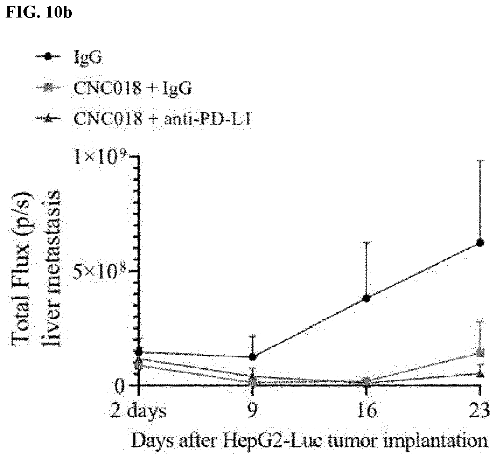

The HepG2-Luc cell line expressing luciferase was prepared, and a mouse model injected with the HepG2-Luc cell line was prepared. Thereafter, IgG, CNC018-IgG, CNC018-anti-PD-L1, CNC018-anti-Asialo, and CNC018 were each administered and the location of the tumor was imaged. The images are shown in FIG. 10a. As a result of checking the amount of total flux that had metastasized to the liver, it could be confirmed that the group co-administered CNC018-anti-PD-L1 had a significant inhibitory effect compared to the control IgG group (FIG. 10b).

As shown in FIG. 10, it could be confirmed that the tumor did not metastasize to the liver in the group co-administered CNC018-anti-PD-L1 compared to the control group, suggesting that the tumor metastasis inhibitory effect of co-administration of CNC018-anti-PD-L1 was remarkable.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention relates to a composition for the highly reliable prevention and treatment of a cancer that is resistant and difficult to treat by a single anticancer therapy and for which there is no effective therapeutic method. Currently, in cancer treatment, patients who respond effectively to immune checkpoint inhibitors differ depending on the type of cancer, and the effect of immune checkpoint inhibitors is also insignificant, and thus the need for new therapeutic compositions is required. The present inventors have developed a composition for preventing or treating cancer using a *Salmonella* strain and an immune checkpoint inhibitor as active ingredients, and have found that tumor growth is significantly inhibited and reduced by the combination therapy of a *Salmonella* strain that selectively acts on cancer and an immune checkpoint inhibitor including PD-L1. Thus, the present invention is expected to provide a fundamental and efficient therapeutic method and composition for cancer.

### Sequence Listing Free Text

SEQ ID NO 1: ClyA protein 
10 20 30 40 50
MIMTGIFAEQ TVEVVKSAIE TADGALDLYN KYLDQVIPWK
TFDETIKELS
60 70 80 90 100
RFKQEYSQEA SVLVGDIKVL LMDSQDKYFE ATQTVYEWCG
VVTQLLSAYI
110 120 130 140 150
LLFDEYNEKK ASAQKDILIR ILDDGVKKLN EAQKSLLTSS
 
QSFNNASGKL
160 170 180 190 200
LALDSQLTND FSEKSSYFQS QVDRIRKEAY AGAAAGIVAG
PFGLIISYSI
210 220 230 240 250
AAGVIEGKLI PELNNRLKTV QNFFTSLSAT VKQANKDIDA
AKLKLATEIA
260 270 280 290 300
AIGEIKTETE TTRFYVDYDD LMLSLLKGAA KKMINTCNEY
QQRHGKKTLF
EVPDV
SEQ ID NO 2: -35 promoter
   TTCGCG
SEQ ID NO 3: -10 promoter
   ATGCATAAT
SEQ ID NO 4: OXB1 promoter

AAGCTGTTGTGACCGCTTGCTCTAGCCAGCTATCGAGTTGTGAACCG
ATCCATCTAGCAATTGGTCTCGATCTAGCGATAGGCTTCGATCTAGCTATGT
AGAAACGCCGTGTGCTCGATCGCCTGACGCTTTTTATCGCAACTCTCTACTGT
TGCTTCAACAGAACATATTGACTATCCGGTATTACCCGGC

## Claims

1. A pharmaceutical composition for preventing or treating cancer, comprising a *Salmonella* strain and an immune checkpoint inhibitor as active ingredients.

2. The pharmaceutical composition of claim 1, wherein the composition is co-administered.

3. The pharmaceutical composition of claim 1, wherein the *Salmonella* strain comprises at least one selected from the group consisting of *Salmonella typhimurium, Salmonella choleraesuis, Salmonella enteritidis, Salmonella dublin, Salmonella. typhisuis, Salmonella derby,* and *Salmonella gallinarum.*

4. The pharmaceutical composition of claim 3, wherein the *Salmonella* strain is *Salmonella typhimurium.*

5. The pharmaceutical composition of claim 1, wherein the immune checkpoint inhibitor comprises at least one selected form the group consisting of cytotoxic T-lymphocyte-associated antigen-4 (CTLA-4), programed cell death protein 1 (PD-1), programmed death-ligand 1 (PD-L1), KIR, LAG3, CD137, OX40, CD47, CD276, CD27, and GITR.

6. The pharmaceutical composition of claim 5, wherein the immune checkpoint inhibitor is programmed death-ligand 1 (PD-L1).

7. The pharmaceutical composition of claim 1, wherein the cancer comprise at least one selected from the group consisting of melanoma, fallopian tube cancer, brain cancer, small intestine cancer, esophagus cancer, lymph node cancer, gallbladder cancer, blood cancer, thyroid cancer, endocrine cancer, oral cancer, liver cancer, biliary tract cancer, colorectal cancer, rectal cancer, cervical cancer, ovarian cancer, kidney cancer, gastric cancer, duodenum cancer, prostate cancer, breast cancer, brain tumor, lung cancer, undifferentiated thyroid cancer, uterine cancer, colon cancer, bladder cancer, ureteral cancer, pancreatic cancer, bone/soft tissue sarcoma, skin cancer, non-Hodgkin lymphoma, Hodgkin lymphoma, multiple myeloma, leukemia, myelodysplastic syndrome, acute lymphoblastic leukemia, acute myeloid leukemia, chronic lymphocytic leukemia, chronic myeloid leukemia, and solitary myeloma.

8. The pharmaceutical composition of claim 1, wherein the composition inhibits cancer growth or cancer metastasis.

9. The pharmaceutical composition of claim 1, wherein the *Salmonella* is *Salmonella* transformed with a vector containing a DNA construct comprising:
a gene encoding a regulatory protein;
a promoter of the gene encoding the regulatory protein;
a first promoter and a second promoter, which are induced by the regulatory protein; and
anyone selected from the group consisting of a gene encoding an anticancer protein downstream of the first promoter and the second promoter, a gene encoding a cytokine, a gene encoding a chemokine, a gene encoding an immune modulator, a cancer antigen-specific oligonucleotide, and a gene encoding a reporter protein.

10. The pharmaceutical composition of claim 9, wherein
the regulatory protein is a TetR protein,
the promoter of the gene encoding the regulatory protein is an OXB1 promoter,
the first promoter, which is induced by the regulatory protein, is a tetA promoter, and
the second promoter, which is induced by the regulatory protein, is a tetR promoter.

11. The pharmaceutical composition of claim 1, wherein the *Salmonella* comprises a DNA construct in which expression of the regulatory protein is regulated by a cis-acting element or a trans-acting element.

12. The pharmaceutical composition of claim 11, wherein the cis-acting element is at least one selected from the group consisting of a ribosome binding site (RBS), a 5'-untransrated region (5'-UTR), a transcription factor binding site, and terminators.

13. The pharmaceutical composition of claim 12, wherein the transcription factor binding site is at least one selected from the group consisting of a promoter of the gene encoding the regulatory protein, an enhancer, and a silencer.

14. The pharmaceutical composition of claim 13, wherein the promoter of the gene encoding the regulatory protein is a weak promoter.

15. The pharmaceutical composition of claim 14, wherein the weak promoter induces a transcript, transcribed from a gene operably linked downstream of the promoter, to be expressed at a level of 1 × 10⁻² or less.

16. The pharmaceutical composition of claim 11, wherein the trans-acting element is at least one selected from the group consisting of a transcription factor, an aptamer, sRNA, and antisense RNA (asRNA).

17. The pharmaceutical composition of claim 9, wherein the anticancer protein is at least one selected from the group consisting of a toxin protein, an antibody specific for a cancer antigen or a fragment of the antibody, a tumor suppressor protein, an angiogenesis inhibitor, a cancer antigen, a prodrug-converting enzyme, and a pro-apoptotic protein.

18. The pharmaceutical composition of claim 17, wherein the toxin protein is at least one selected from the group consisting of ricin, saporin, gelonin, momordin, debouganin, diphtheria toxin, Pseudomonas toxin, hemolysin (HlyA), FAS ligand (FASL), tumor necrosis factor-α (TNF-α), TNF-related apoptosis-inducing ligand (TRAIL), and cytolysin A (ClyA).

19. The pharmaceutical composition of claim 17, wherein the tumor suppressor protein is at least one selected from the group consisting of retinoblastoma (RB) protein, p53 protein, adenomatous polyposis coli (APC) protein, phosphatase and tensin homologue (PTEN) protein, and cyclin dependent kinase inhibitor 2A (CDKN2A) protein.

20. The pharmaceutical composition of claim 17, wherein the angiogenesis inhibitor is at least one selected from the group consisting of angiostatin, endostatin, thrombospondin, and protease inhibitory proteins.

21. A method of preventing or treating cancer by administering to a subject a pharmaceutical composition comprising a *Salmonella* strain and an immune checkpoint inhibitor.

22. Use of a *Salmonella* strain and an immune checkpoint inhibitor for preventing or treating cancer.
